# EUROPEAN PATENT SPECIFICATION

(11) **EP 4 188 504 B1**
(45) Date of publication and mention of the grant of the patent: **20.05.2026**
(21) Application number: 21849924.2
(22) Date of filing: 27.07.2021
(51) Int. Cl.: A61M 25/00, A61M 25/01

(54) **EMBOLIZATION MICROCATHETER FOR DELIVERY OF BEADS TO PERIPHERAL VESSELS**
EMBOLISATIONSMIKROKATHETER ZUR ABGABE VON KÜGELCHEN AN PERIPHERE GEFÄSSE
MICROCATHÉTER D'EMBOLISATION POUR L'ADMINISTRATION DE BILLES À DES VAISSEAUX PÉRIPHÉRIQUES

(30) Priority: 30.07.2020 IL 27641820
(43) Date of publication of application: 07.06.2023
(73) Proprietor: Accurate Medical Therapeutics Ltd., 7608802 Rehovot (IL)
(72) Inventor: DAGAN, Tom, 8496500 Omer (IL); ZIPORY, Yuval, 7175472 Modiin (IL); HARBATER, Osnat, 4358142 Raanana (IL); MILLER, Eran, 7680300 Moshav Beit Elazari (IL)
(74) Representative: AWA Denmark A/S
(86) International application number: PCT/IL2021/050905
(87) International publication number: WO 2022/024118

(56) References cited:
- WO-A1-2019/087191
- WO-A1-2019/087191
- US-A1- 2015 005 801
- US-A1- 2019 217 052
- US-A1- 2019 217 052
- US-A1- 2020 230 355
- US-B2- 7 507 229
- US-B2- 7 507 229

## Description

### TECHNICAL FIELD

The present disclosure generally relates to the field of microcatheters for embolization, specifically to embolization catheters suitable for delivery of embolization beads to peripheral blood vessels.

### BACKGROUND

Transarterial embolization therapy, tumor embolization, or transcatheter arterial embolization (TAE), involves administration of embolization material (which may include chemotherapeutics or/and radiotherapeutics) directly to a tumor (for example, liver tumors), via a microcatheter.

Embolization of tumors is typically performed utilizing microcatheters due to the requirement for selectively affecting the tumor while preventing, as much as possible, damage to healthy tissue. A major problem associated with embolization is "non-target embolization", where the embolic material travels to blood vessels, other than those directly feeding the target tumor or tumor region, thus damaging healthy tissues, resulting in unpleasant and even hazardous outcomes.

During embolization, especially of peripheral vessels, the embolization catheter must be advanced through small and often tortuous vessels. Accessibility to these vessels is difficult, if not precluded, using large and/or stiff microcatheters. Moreover, blood vessels in the body tend to go into spasm when manipulated, causing an ineffective embolic material delivery, so flexible micro-sized catheters are an absolute necessity.

A major drawback of trans-catheter embolization is that the embolization material, which is typically invisible, can be refluxed and reach non-target tissue and cause damage to them. In addition, reflux of embolization material may negatively affect the delivery of the embolization material to the target tissue, and thus impair treatment effectiveness and its clinical outcome.

U.S. Patent No. 7,507,229B2 describes a multi-layer catheter for navigation to remote locations within the body, comprising a liner layer, a braid layer and an outer jacket.

U.S. Patent Application Publication No. 2019/217052A1 describes an embolization microcatheter having an elongated microcatheter body configured for passing therein a suspension of particles suspended in a suspension fluid; and a microcatheter head connected to a distal end of said microcatheter body and comprising a proximal head section and a distal head section, wherein the proximal head section includes a proximal wall forming a proximal section lumen, the proximal wall having a plurality of through-holes, wherein the distal head section includes a distal wall forming a distal section lumen, and a suspension delivery opening at a distal end of the distal end section; wherein the distal section is devoid of through-holes, and wherein each of the plurality of through-holes are shaped and/or sized to allow passage therethrough of the suspension fluid while blocking passage of the particles suspended therein.

International patent application publication No. WO 2019/087191 A1 describes an embolization microcatheter configured to deliver embolization particles to a target area and minimize or prevent embolization of a non-target area. The microcatheter includes a section, located between the distal and proximal ends of the microcatheter, having a skeleton formed of braided wires and a polymeric layer intercalated into and/or overlaying the skeleton. This section includes a plurality of axial slits, each slit having a smallest cross-sectional dimension configured to prevent outflow of the embolization particles.

U.S. Patent Application Publication No. 2020/230355 describes a microcatheter comprising an inner layer, a strike layer and an outer layer and a braided skeleton located between the inner layer and the outer layer, wherein the inner layer is made of Polytetrafluoroethylene (PTFE) and has a thickness of 0.0015 inch or less, wherein the strike layer includes a polyether block amide and has a thickness of 0.001 inch or less, and wherein a distal portion of said outer layer is made of polycarbonate-based thermoplastic polyurethane having a shore of 90A or below.

U.S. Patent Application Publication No. 2015/005801 describes a system for use in a vascular procedure includes a guidewire dimensioned to remotely access a neurovascular space, a microcatheter for positioning over the guidewire, an interventional treatment element for passage within the catheter member of the microcatheter, and an outer guide positionable over the catheter member of the microcatheter upon removal of the catheter hub. The microcatheter includes an elongated catheter member and a catheter hub. The catheter member defines a longitudinal axis and has a longitudinal length and with proximal and distal ends. The catheter hub is connected to the proximal end of the catheter member and is dimensioned and adapted to be selectively released from the catheter member. The outer guide is advanceable over the catheter member of the microcatheter after the catheter hub is released from the catheter member to a location proximate the lesion. The interventional treatment element is adapted to perform treatment on the lesion within the vasculature.

Microcatheters with filter sections for delivery of embolization beads, while preventing backflow of the beads, have been disclosed by the inventors of the present applications. However, there remains a need for microcatheters capable of delivering embolization beads to peripheral blood vessels, i.e. microcatheters with a sufficiently small outer diameter for unhindered entry into peripheral blood vessels yet which do not cause clogging or kinking of the microcatheter.

### SUMMARY OF THE INVENTION

The present disclosure relates to an embolization microcatheter according to claim 1. Advantageous embodiments are disclosed in the dependent claims.

In this disclosure the following non-SI units are used, which may be converted to the respective SI or metric unit according to the following conversion factors or formulas: 1 Pound per square inch (psi) = 6894.76 Pascal; 1 inch = 0.0254 m. The present disclosure relates to embolization microcatheters which are suitable for passing through and/or reaching peripheral arteries (typically 1.7 or 1.9 French (Fr)), while also facilitating unhindered delivery of embolization beads.

This is advantageously achieved by the unique structure of the wall of the embolization microcatheters which has an outer diameter of less than 0.8 mm, yet is still trackable and torque resistant. The large majority of the wall is made of a braid, a polymer formed around the braid and an inner liner coating an inner surface of the wall. The distal tip of the microcatheter (approximately the last 1 mm - 1.5 mm) includes a radiopaque marker for positioning. The distal tip is further characterized by its wall being devoid of the braid. This may, according to some embodiments, advantageously compensate for the increased wall thickness caused by the radiopaque marker.

The herein disclosed embolization microcatheter further includes a filter section with a plurality of openings configured for outflow of fluids while preventing outflow of the beads, thus providing concentrated delivery of the embolization beads through the distal end opening of the microcatheter, while ensuring minimal backflow. Advantageously, the size, shape and distribution of the opening enables smooth delivery of the beads, despite the small diameter of the microcatheter. According to some embodiments, the plurality of side openings is distributed on at least five circumferential rings spaced apart from each other by 100 microns - 200 microns.

According to some embodiments, the wall of the tubular member includes a multiplicity of sections, each having a length of 5 mm -150 mm and each formed of a different polymer. This may advantageously on the one hand provide pushability and on the other hand provide efficient maneuvering through tortuous blood vessels.

According to some embodiments, there is provided an embolization microcatheter for embolization of peripheral arteries comprising: an elongated tubular member comprising a distal end extending from a proximal marker to a distal end opening, the distal end having an outer diameter of about 0.7 mm or less, wherein the elongated tubular member comprises a multiplicity sections, each having a length of 5 mm -120 mm, wherein a wall of each of the multiplicity of sections comprises a braid, a polymer formed around the braid and an inner liner coating an inner surface thereof; wherein the polymer of at least some of the multiplicity sections differ and wherein a thickness of the wall is less than 100 microns; a distal tip having a length of 0.5 mm - 3 mm and extending between a proximal end of a distal radiopaque marker of the elongated tubular member and a distal end opening of the elongated tubular member; and wherein the wall of the distal tip is devoid of braid.

According to some embodiments, the inner lumen of the distal end is in a range of 0.3 mm - 0.7 mm. According to some embodiments, the inner lumen of the distal end is in a range of 0.35 mm - 0.55 mm. According to some embodiments, the inner lumen of the distal tip is below 0.5 mm.

According to some embodiments, the thickness of the wall is less than 90 microns.

According to some embodiments, the braid is made of tungsten wires. According to some embodiments, the braid is made of wires having a diameter of 15-20 microns. According to some embodiments, the braid has a picks per inch (PPI) of 150-220.

According to some embodiments, the multiplicity of sections comprises at least 5 sections. According to some embodiments, the multiplicity of sections comprises at least 9 sections. According to some embodiments, the distal most section of the multiplicity of sections has a length of 5-15 mm.

According to some embodiments, the embolization microcatheter further comprises a distal radiopaque marker, wherein the distal radiopaque marker comprises a metal marker band.

According to some embodiments, the distal most section of the multiplicity sections comprises a filter formed in the wall of the elongated tubular member, the filter comprising a plurality of side openings, the plurality of side opening distributed in at least 5 circumferential rings spaced apart from each other by 100 microns - 200 microns.

According to some embodiments, the plurality of openings is in the form of axial slits. According to some embodiments, the axial slits have a length of about 100-150 microns and a height of about 20-40 microns.

According to some embodiments, the distal most of the circumferential rings of the distal most filter section is positioned about 2-6 mm proximally to the distal end opening.

According to some embodiments, the plurality of side opening is distributed in at least 8 circumferential rings. According to some embodiments, each of the circumferential rings comprises 4-8 axial slits. According to some embodiments, the proximal most circumferential ring of the at least 5 circumferential rings comprises fewer side openings than rings distal thereto. According to some embodiments, the proximal most circumferential ring comprises 1-3 side openings. According to some embodiments, the side openings, of the proximal most circumferential section, is circumferentially shifted relative to the side openings in its neighboring circumferential section.

According to some embodiments, there is provided an elongated tubular member terminating with a distal end opening, the elongated tubular member comprising: a distal end extending 200-500 mm from the distal end opening toward a proximal end of the elongated tubular member, wherein an outer diameter of the elongated tubular member is less than 0.8 mm, wherein the elongated tubular member comprises a multiplicity sections, each having a length of 5 mm - 150 mm, wherein a wall of each of the multiplicity of sections comprises a braid, a polymer formed around the braid and an inner liner coating an inner surface thereof; wherein the polymer of the multiplicity sections differ, wherein a thickness of the wall is less than 100 microns, and wherein the braid is made of wires having a diameter of 15-18 microns and has a picks per inch (PPI) of 200-350

According to some embodiments, the inner lumen of the distal end is in a range of 0.35 mm - 0.6 mm. According to some embodiments, the outer diameter of the distal end of the elongated tubular member is below 0.75 mm.

According to some embodiments, the thickness of the wall is less than 90 microns.

According to some embodiments, the braid is made of tungsten wires.

According to some embodiments, the multiplicity of sections comprises at least 5 sections. According to some embodiments, the multiplicity of sections comprises at least 9 sections. According to some embodiments, the distal most section of the multiplicity of sections has a length of 10-20 mm.

According to some embodiments, the embolization microcatheter further comprises a radiopaque marker comprises According to some embodiments, the radiopaque marker comprises a metal marker band.

According to some embodiments, the distal most section of the multiplicity sections comprises a filter formed in the wall of the elongated tubular member, the filter comprising a plurality of side openings, the plurality of side opening distributed in at least 5 circumferential rings spaced apart from each other by 100 microns - 200 microns.

According to some embodiments, the plurality of openings is in the form of axial slits. According to some embodiments, the axial slits have a length of about 100-150 microns and a height of about 20-40 microns.

According to some embodiments, the distal most of the circumferential ring of the distal most filter section is positioned about 2-6 mm proximally to the distal end opening.

According to some embodiments, the plurality of side opening is distributed in at least 8 circumferential rings. According to some embodiments, each of the circumferential rings comprises 4-8 axial slits.

According to some embodiments, the proximal most circumferential ring of the at least 5 circumferential rings comprises fewer side openings than rings distal thereto. According to some embodiments, the proximal most circumferential ring comprises 1-3 side openings. According to some embodiments, the side openings, of the proximal most circumferential section, is circumferentially shifted relative to the side openings in its neighboring circumferential section.

Certain embodiments of the present disclosure may include some, all, or none of the above characteristics. One or more technical advantages may be readily apparent to those skilled in the art from the figures, descriptions and claims included herein. Moreover, while specific characteristics have been enumerated above, various embodiments may include all, some or none of the enumerated characteristics.

In addition to the exemplary aspects and embodiments described above, further aspects and embodiments will be further expanded upon in the figures and the following detailed descriptions.

### BRIEF DESCRIPTION OF THE FIGURES

The features, nature and advantages of the present disclosure will become more apparent from the detailed description set forth below when taken in conjunction with the drawings in which like reference characters identify correspondingly throughout. Identical structures elements or parts that appear in more than one figure are generally labeled with the same number in all the figures in which they appear. Alternatively, elements or parts that appear in more than one figure may be labeled with different numbers in the different figures in which they appear. The dimensions of the components and features in the figures were chosen for convenience and clarity of presentation and are not necessarily shown to scale. The figures are listed below.
**FIG. 1A** schematically illustrates a microcatheter comprising an outer layer including a plurality of sections, the plurality of sections made of different polymeric materials, according to some embodiments.
**FIG. 1B** schematically illustrates a perspective, cutaway view of the distal end of the microcatheter of **FIG. 1A** illustrating the outer layer, the strike layer, the inner layer, the braided skeleton located between the inner layer and the outer layer.
**FIG. 2A** schematically illustrates an embolization microcatheter with a fluid barrier forming section, according to some embodiments.
**FIG. 2B** schematically illustrates a magnified and partially exposed view of the distal end of the microcatheter of **FIG. 2A****,** according to some embodiments.
**FIG. 2C** schematically illustrates a magnified and partially exposed view of the distal tip of the microcatheter of **FIG. 2A****,** according to some embodiments.
**FIG. 2D** schematically illustrates a slit formed by selective cutting through the wall of a fluid barrier forming section, such as the fluid barrier forming section of the embolization microcatheter of **FIG. 2A****,** according to some embodiments.
**FIG. 3** schematically illustrates an optional slit pattern for an embolization microcatheter such as the embolization microcatheter of **FIG. 2A****,** according to some embodiments.
**FIG. 4** schematically illustrates another optional slit pattern for an embolization microcatheter, such as the embolization microcatheter of **FIG. 2A****,** according to some embodiments.

### DETAILED DESCRIPTION OF THE INVENTION

The detailed description set forth below, in connection with the appended drawings, is intended as a description of various configurations and is not intended to represent the only configurations in which the concepts described herein may be practiced. The detailed description includes specific details for the purpose of providing a thorough understanding of the various concepts. However, it will also be apparent to one skilled in the art that these concepts may be practiced without specific details being presented herein. In some instances, well-known features may be omitted or simplified in order to avoid obscuring the disclosure.

One of the main challenges of embolization microcatheters is ensuring a small enough outer diameter to facilitate entry into peripheral vessels, while also ensuring unhindered delivery of embolization beads (including unhindered flow within the lumen of the microcatheter) as well as a catheter wall which is strong, trackable and kink resistant.

Advantageously, these requirements are met by the herein disclosed microcatheter and the structural characteristics of its tubular wall.

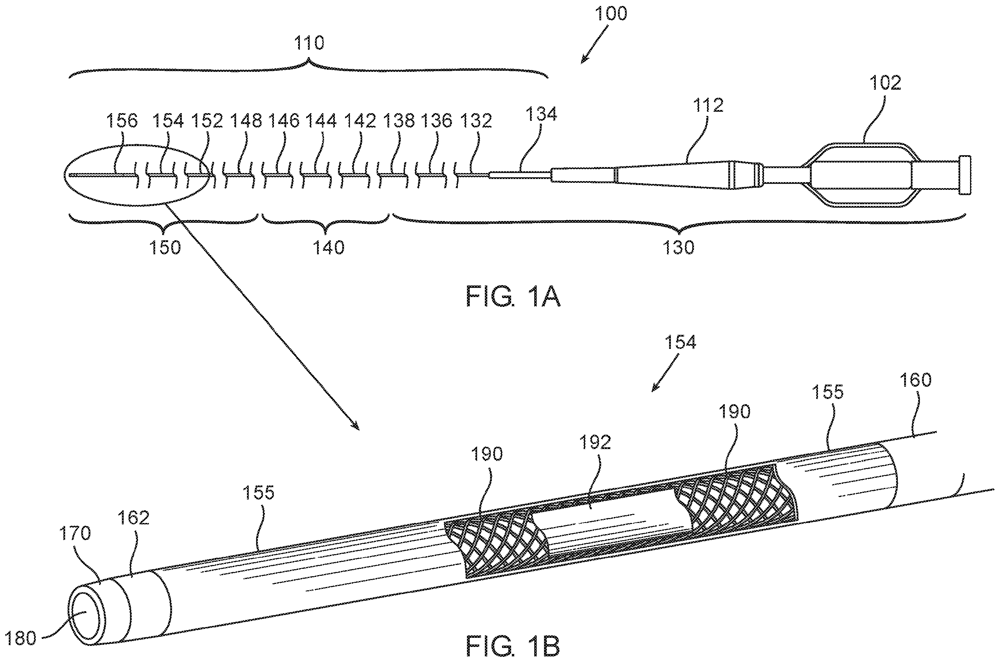

Reference is now made to **FIG. 1A,** and **FIG. 1B****,** which schematically illustrate an embolization microcatheter **100** and magnified/exposed views of a distal part thereof.

As used herein, the terms "embolization", "transcatheter embolization", "transcatheter arterial embolization" and "TAE" may be used interchangeably and refer to the passage and lodging of an embolus within the bloodstream for therapeutic purposes, for example, as a hemostatic treatment of bleeding or as a treatment for some types of cancer by deliberately blocking blood vessels to starve the tumor cells.

Embolization microcatheter **100** includes an elongated tubular member **110.** The proximal end **130** of microcatheter **100** includes a hub **102** which is molded on or otherwise attached to elongated tubular member **110** of microcatheter **100.**

Hub **102** is configured to allow access to the lumen of elongated tubular member **110** for a variety of functions, such as the injection of fluids or drugs, or the introduction of guidewires. Hub **102** optionally includes a strain relief **112,** preferably mechanically coupled to hub **102.** Strain relief **112** may be made of a polymeric material and may, as illustrated, be tapered at its distal end. Strain relief **112** and be configured to provide structural support to elongated tubular member **110,** to prevent it from kinking.

According to some embodiments, the wall of elongated tubular member **110** may include a plurality of sections, each section characterized by the polymers utilized. According to some embodiments, a plurality of sections may include 3, 4, 5, 6, 7, 8, 9, 10, 15, 20 or more sections. Each possibility is a separate embodiment.

According to some embodiments, the different polymeric layers may contribute to different characteristics of the layer/section and thus of elongated tubular member **110.** For example, the different polymeric layers may contribute to the elasticity, flexibility, stretch-ability, strength, hardness, rigidity, ultimate tensile strength, elongation or any other characteristic of the layer and thus the microcatheter. Each possibility is a separate embodiment.

The proximal **130** end of elongated tubular member **110,** attached to strain relief **112,** includes first section **132.** The outer layer of section **132** may be made of a polymeric material having a relatively high hardness such as a polyether block amide having a hardness of about 70D shore and/or a flexural modulus of about 74,000 psi. According to some embodiments, proximal end **132** may have a length of 600-1300mm (e.g. about 1000 mm).

Optionally, part of section **132** may include a heat shrink material **134** covering the joint between strain relief **112** and elongated tubular member **110.**

Adjacent section **132,** is a second section, section **136,** which is slightly softer. The outer layer of section **132** may be made of polymeric material having a hardness of about 60D-70D shore and/or a flexural modulus between 41,000 psi-74,000 psi. Section **136** may have a length of 10-40 mm, or 20-30 mm e.g. 25 mm. Section **136** may be followed with a slightly softer section, section **138,** which may be made of polymeric material having a hardness about 60-65D shore and/or a flexural modulus of about 41,000 psi. Section **138** may have a length of 60-80 mm, e.g. 70 mm.

According to some embodiments, the polymeric material of section **138** is softer than that of section **136.** According to some embodiments, the polymeric material of section **136** is softer than that of section **132.**

Intermediate part **140** of elongated tubular member **110** includes section **142** having an outer layer which may be made of a polyether block amide or other suitable polymer having a hardness of about 55D shore and/or a flexural modulus of about 25,000 psi, section **144** having an outer layer made of a polymeric material having a hardness of about 55D shore such as a polycarbonate-based thermoplastic urethane having a hardness of about 50D shore, section **146** having an outer layer made of a one or more polycarbonate-based thermoplastic urethanes having a hardness between 55D shore and 95A shore, and section **148** having an outer layer made of a one or more polycarbonate-based thermoplastic urethanes having a hardness of 95A shore. Section **142** may have a length of 50-90 mm, e.g. 70 mm. Section **144,** may have a length of 80-1100 mm (e.g. about 90 mm). Section **146** may have a length of 50-70 mm (e.g. about 65 mm). Section **148** may have a length of 5-30 mm (e.g. about 15 mm).

According to some embodiments, the polymeric material of section **148** is softer than that of section **146.** According to some embodiments, the polymeric material of section **146** is softer than that of section **144.** According to some embodiments, the polymeric material of section **144** is softer than that of section **142.**

Distal end **150** of elongated tubular member **110** includes sections **152** having an outer layer made of a polymeric material having a hardness of about 95A shore, such as, for example, a polycarbonate-based thermoplastic urethane having a hardness of 95A shore and a length of 10-40 mm (e.g. about 25 mm) and **154** having an outer layer made of a polymeric material having a hardness of about 85A shore such as, for example, a polycarbonate-based thermoplastic urethane having a hardness of about 85A and a length of about 3-10 mm (e.g. about 6 mm). The polymer of section **154** further includes a polymeric marker such as, but not limited to, tantalum powder and section **156** having an outer layer made of a polymeric material having a hardness of about 80A shore such as a polycarbonate-based thermoplastic urethane having a hardness of about 85A shore having a length of about 5-15 mm (e.g. about 9.5 mm).

According to some embodiments, elongated tubular member **110** may have an outer diameter in the range of 0.5 mm-1.5 mm or in the range of 0.55mm - 1.0 mm. According to some embodiments the outer diameter of tubular member **110** may vary from its proximal end to its distal end. According to some embodiments, the outer diameter of tubular member **110** may be gradually decreasing from its proximal end to its distal end.

According to some embodiments, the outer diameter of the proximal end of tubular member **110,** including section **132,** may be in the range of 0.8-1.0 mm, such as, but not limited to, about 0.95 mm. According to some embodiments, the outer diameter of intermediate section of the tubular member **110** may be in the range of 0.7-0.9 mm. According to some embodiments, the outer diameter of the tubular member may decrease from about 0.9 mm to about 0.7 mm towards the distal end. According to some embodiments, the outer diameter of distal end of tubular member **110** may be in the range of 0.5-0.75 mm. According to some embodiments, the outer diameter of the tubular member may decrease from about 0.75 mm to about 0.55 mm towards the distal end opening. According to some embodiments, the outer diameter of distal tip **170** may be in the range of 0.55-0.65 mm. According to some embodiments, the outer diameter of distal tip **170** may be about 0.56 mm along the entire length thereof except over marker band **162** over which the outer diameter may be about 0.6-0.65 mm.

According to some embodiments, the outer diameter of section **156** is smaller than the outer diameter of section **154.** According to some embodiments, the outer diameter of section **154** is smaller than the outer diameter of section **152.** According to some embodiments, the outer diameter of section **152** is smaller than the outer diameter of section **148.** According to some embodiments, the outer diameter of sections **148** and **146** is smaller than the outer diameter of section **144.** According to some embodiments, the outer diameter of sections **144** is smaller than the outer diameter of section **142.** According to some embodiments, the outer diameter of sections **142** is smaller than the outer diameter of section **136** and **132.**

According to some embodiments, the outer diameter is essentially constant along a same section.

According to some embodiments, elongated tubular member **110** may have an inner diameter in the range of 0.35-0.65 or 0.4-0.60 mm. According to some embodiments, the inner diameter may be larger at the proximal end than at the distal end. According to some embodiments, the inner diameter of elongated tubular member **110** may be about 0.55 mm along the entire length of elongated tubular member **110** except the distal tip **170** (encompassing the last about 5-15 mm of the microcatheter, e.g. the last 10 mm), which may have an inner diameter of about 0.35-0.50 mm (e.g. about) 0.42 mm. According to some embodiments, the section extending from distal tip **170** to about 60-90 mm (e.g. about 70 mm) proximal thereto may be tapered.

As used herein, the term "distal end opening" refers to the end opening of the microcatheter leading into the lumen thereof. According to some embodiments, distal end opening **180** defines the termination of the microcatheter. According to some embodiments, distal end opening **180** may have an inner diameter essentially equal to the inner diameter of the microcatheter lumen. According to some embodiments, the distal end opening **180** may have an inner diameter which is smaller than the inner diameter of the microcatheter lumen leading to a narrowing of the lumen toward the end thereof.

Distal end **150** of elongated tubular member **110** may include a proximal marker **160** and a distal marker **162** (also seen in **FIG. 1B****).** According to some embodiments, proximal marker **160** may be a radiopaque powder embedded the outer layers, as described herein above with regards to section **154.** According to some embodiments, proximal marker **160** may be positioned approximately 5-20 or 10-15 mm from the distal end opening **180.** According to some embodiments, distal marker **162** may be a radiopaque alloy submerged in outer layer of distal tip **170.** According to some embodiments, distal marker **162** may be positioned approximately 0.25-1 mm proximally from distal end opening **180.**

Reference is now made to **FIG. 1B** which schematically illustrates a perspective, cutaway view of the distal part of distal end **150** of microcatheter **100** shown in **FIG. 1A** extending from proximal marker **160** to distal end opening **180.** As seen from the exploded view, underneath outer layer **155** is a braid **190.**

According to some embodiments, braid **190** extends along the entire length of shaft elongated tubular member **110.** Alternatively, braid **190** extends along the entire length of elongated tubular member **110,** apart from distal tip **170.** According to some embodiments, braid **190** may extend from the proximal end of tubular member **110** until distal marker **162.** According to some embodiments, the part of tubular member **110** extending from distal marker **162** to distal end opening **180** may be devoid of braid.

Preferably, braid **190** has a picks per inch (PPI) ensuring that, in combination with a low durometer polymer, a flexible distal end is obtained, and in combination with a polymer having a higher durometer a relatively stiff proximal end is provided.

According to some embodiments, braid **190** may be made of a plurality of wires.

As used herein the terms "braid" and "braided skeleton" may refer to a structural element, such as a tubal element formed of a plurality of interlaced wires. According to some embodiments, the braid may be formed of at least three interlaced wires forming a tube. According to some embodiments, the braid may include 8-48 wires or 12-32 wires. Each possibility is a separate embodiment. As a non-limiting example, the braid may include 16 wires.

According to some embodiments, the wires forming the braid may have a diameter in the range of 10-40 microns or 12-20 microns or 15-18 microns or any other suitable diameter within the range of 10-60 microns. Each possibility is a separate embodiment. As a non-limiting example, the wires forming the braid may have a diameter of 18 microns.

According to some embodiments, the braid may be made from tungsten, stainless steel, Nickel titanium (also referred to as Nitinol), nitinol, cobalt chrome, platinum iridium, nylon or any combination thereof. Each possibility is a separate embodiment. As a non-limiting example, the wires may be tungsten wires.

According to some embodiments, at least some of the wires forming the braided skeleton may be braided in a same or opposite direction, i.e. left/right handed. Advantageously, the braiding structure allows good torque-ability (better than a coiled skeleton), low flexural rigidity (i.e. good flexibility), good push-ability (better than a coiled skeleton), and superior kink-resistance.

According to some embodiments, at least some of the wires forming the braided skeleton may be non-circular/round.

According to some embodiments, the braided skeleton may have a wire arrangement of 100-400 picks per inch (PPI), 150-375 PPI or 200-350 PPI. Each possibility is a separate embodiment. As a non-limiting example, the braided skeleton may have a wire arrangement of about 250 PPI. As another non-limiting example, the braided skeleton may have a wire arrangement of about 275 PPI. As another non-limiting example, the braided skeleton may have a wire arrangement of about 300 PPI. As another non-limiting example, the braided skeleton may have a wire arrangement of about 325 PPI. As another non-limiting example, the braided skeleton may have a wire arrangement of about 350 PPI. Those skilled in the art will appreciate the term picks per inch (PPI) is a measurement of braid wire density and represents the number of picks (e.g. weft wires) per inch of braid.

According to some embodiments, the PPI of the braid may differ along the length of tubular member **110.** According to some embodiments, the PPI at the distal tip is higher than at sections distal thereto. As a nonlimiting example, the PPI of braid **190** at the distal tip may be about 200 PPI whereas the PPI of sections proximal thereto may be about 160 PPI. According to some embodiments, braid **190** includes a transition zone at which the PPI of the braid transition is from 200 PPI to 160 PPI.

Underneath braid **190** is an inner liner **192,** which may be made of Polytetrafluoroethylene (PTFE). According to some embodiments, inner liner **192** may have a thickness of about 5-25 microns or 5-15 microns. Each possibility is a separate embodiment. According to some embodiments, the liner may be ram-extruded liner.

According to some embodiments, the total thickness of the wall of the distal end of the elongated tubular member does not exceed about 100 microns. According to some embodiments, the total thickness of the wall of the distal end of the elongated tubular member does not exceed about 90 microns. According to some embodiments, the total thickness of the wall of the distal end of the elongated tubular member does not exceed about 80 microns.

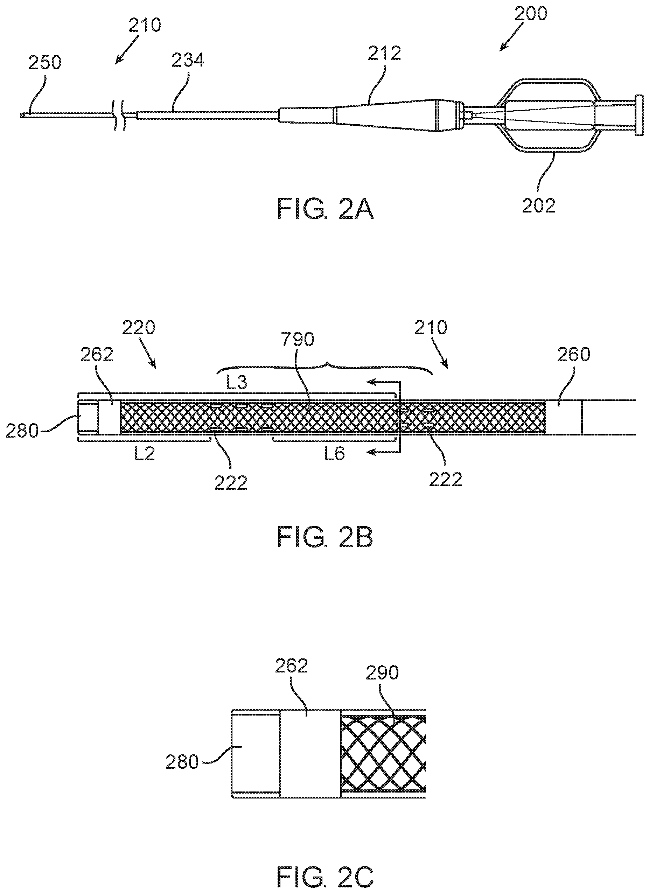

Reference is now made to **FIG. 2A****-****FIG. 2D****,** which schematically illustrate an embolization microcatheter **200** and magnified/exposed views of parts thereof. Embolization microcatheter **200** may be similar to embolization microcatheter **100** apart from embolization microcatheter **200** also including a filter **220.**

The proximal end of microcatheter **200** includes a hub **202** which is molded on or otherwise attached to microcatheter **200.** Hub **202** is configured to allow access to the lumen of microcatheter **100** for a variety of functions, such as the injection of fluids or drugs, or the introduction of guidewires. Hub **202** includes a strain relief **212,** preferably mechanically coupled to hub **202.** Strain relief **212** may be made of a polymeric material and may, as illustrated, be tapered at its distal end. Strain relief **212** is configured to provide structural support to microcatheter **200,** thereby preventing/minimizing kinking of microcatheter **200.**

Reference is now made to **FIG. 2B** which schematically illustrates a partially exposed view of distal end **250** of microcatheter **200** shown (the portion of the distal end **250** extending between proximal marker **260** and distal marker **262** being exposed). Similarly to elongated tubular member **110,** underneath the outer layer is a braid **290,** which is essentially the same as braid **190.**

Filter **220** including with a plurality of penetrating side openings formed in the wall of elongated tubular member **210,** schematically illustrated in **FIG. 2B****.**

As used herein, the term "plurality" with referral to the side openings refer to 2 or more, 3 or more, 5 or more, 10 or more, 15 or more, 20 or more or 25 or more axial slits. Each possibility is a separate embodiment

According to some embodiments, the filter **220** may be an integral part of elongated tubular member **110** and may extend along a length of 0.3mm-20mm, such as 1mm-10mm, 1mm-5mm, 1.5mm-5 mm, 2mm-5mm or any other in-between suitable length. Each possibility is a separate embodiment.

According to some embodiments, filter **220** may have a total open area, formed by the side openings, in the range of 0.2-1mm², 0.2-0.6mm², 0.3-1mm2, 0.3-0.5mm², 0.4-0.6mm², 0.5-1.5mm², 1.0-3.5mm², 1.5-4mm², 2.0-3.5mm² or any other suitable area within the range of 0.1-4mm². Each possibility is a separate embodiment. According to some embodiments, at least 5%, at least 10%, at least 15% of filter **220** is open area formed by the side openings. According to some embodiments, 5%-30%, at least 7%-25%, 7%-20%, 5%-15% of filter **220** is open area formed by the side openings. Each possibility is a separate embodiment.

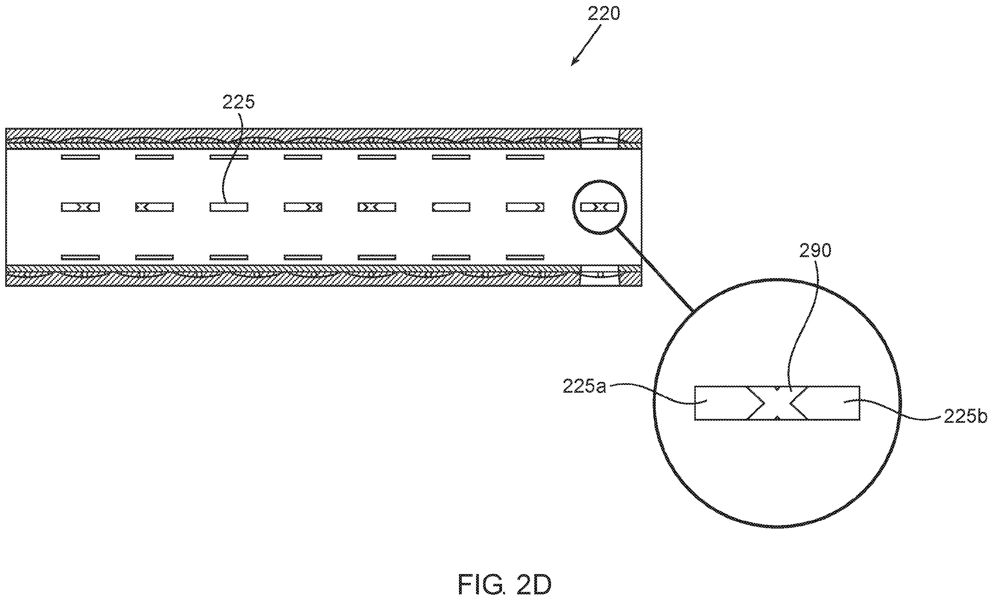

According to some embodiments, side openings **225** may be formed by selective cutting (e.g. selective laser cutting), that is, without cutting the wires forming braid **290** as illustrated in **FIG. 2D****.** According to some embodiments, the part of the liner positioned below the wires remains intact. According to some embodiments, both the polymeric layer and the inner liner positioned between the wires of braid **290** are penetrated when forming the slits. Advantageously, the selective cutting of the polymeric layer (leaving braid **290** essentially intact may provide subdivision of at least some of the side-openings into two or more sub-side-openings (illustratively depicted as side opening **225a** and **292b)** separated by the braid but not by the polymeric outer layer.

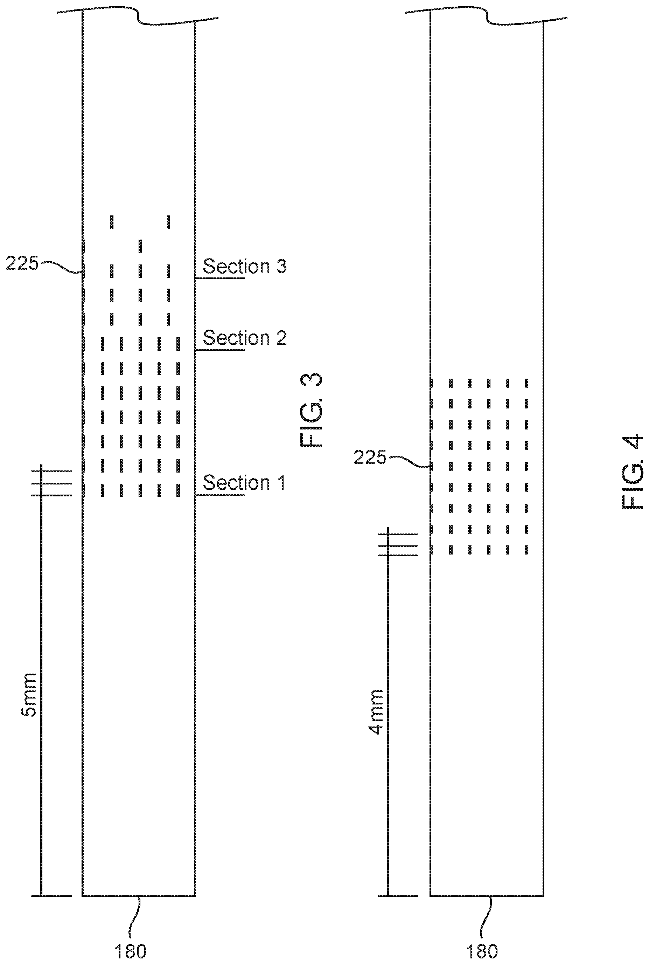

One optional structure of filter **220** is provided in **FIG. 3****.** According to some embodiments, the structure is suitable for a 1.9 Fr embolization microcatheter. As seen in **FIG. 3****,** filter **220** may include three filter sections, each filter section comprising a plurality of side openings **225,** distributed in circumferential rings around elongated filter **220.**

According to some embodiments, filter section 1 may include 1-10 or 2-8 or 4-7 rings (here illustrated as 7 rings) of side openings. According to some embodiments, each of the rings may include 1-8 side openings or 2-6 side openings, such as, but not limited to 6 side openings per ring. According to some embodiments, filter section **221** may include a total of 20-50 or 25-60 side openings, such as but not limited to 42 side openings. According to some embodiments, the distal most of the rings of filter section 1 may be positioned about 3-10 mm or 4-8 mm, such as but not limited to about 5 mm from the distal end opening **180.**

According to some embodiments, filter section 2 may include 1-5 or 2-4 rings of side openings, such as, but not limited to 3 rings of side opening. According to some embodiments, each of the rings may include 1-6 side openings or 2-4 side openings, such as, but not limited to 4 side openings per ring. According to some embodiments, filter section 2 may include a total of 5-20, 6-16 side openings, such as but not limited to 12 side openings.

According to some embodiments, side openings of filter section 2 may be circumferentially shifted relative to side openings of filter section 1.

According to some embodiments, filter section 3 may include 1-5 or 2-4 rings of side openings, such as, but not limited to 2 rings of side opening. According to some embodiments, each of the rings may include 1-4 side openings or 1-3 side openings per ring, such as but not limited to 2 side openings per ring. According to some embodiments, filter section 3 may include a total of 2-6, or 2-4 side openings, such as but not limited to 4 side openings.

According to some embodiments, the side openings of a first ring in section 3 may be circumferentially shifted relative to side openings of a second ring in section 3. According to some embodiments, side openings of filter section 3 may be circumferentially shifted relative to side openings of filter section 1.

According to some embodiments, side openings **225** may have a dimension of about 150x25 microns, about 150x30 microns, about 125x30 microns or about 100x30 microns.

According to some embodiments, each ring of side openings of filter sections 1-3 may be spaced apart from its neighboring ring by 100-200 microns or by 120-180 microns, such as but not limited to 150 microns.

Advantageously, filter **220** may be formed by selective cutting of the polymeric layer (leaving braid **290** essentially intact). According to some embodiments, at least some of side openings **225** may include sub-side-openings (such as sub-side-opening **225a** and **225b** in **FIG. 2D****)** separated by braid **290,** but not by the polymeric outer layer.

According to some embodiments, the slits may be positioned at a same or a different longitudinal position. Each possibility is a separate embodiment. According to some embodiments the distribution of the slits may be staggered, zig-zagged or any other suitable even or uneven distribution.

Advantageously, the filter **220** may be configured for kink-free bending despite the plurality of side openings formed in the wall thereof. According to some embodiments, the flexibility of the filter **220** is determined by the number of side openings, their minimal cross-sectional dimension, their width, length spacing, geometry, distance from distal outlet etc., as essentially described herein, may enable kink-free bending thereof.

As used herein the term "kink-free bending" may refer to a bending of filter **220,** which does impede flow therethrough. According to some embodiments, filter **220** may be configured for kink-free bending at an angle of about 180 degrees. According to some embodiments, filter **220** may be configured for kink-free bending at a minimum bending radius in the range of about 0.5 to 1.5 mm, for example 0.5 to 1.2, 0.5 to 1 mm, or any radius in-between.

Advantageously, microcatheter **200** including filter **220** provides effective reflux prevention, which requires a relatively high density of side openings, while a small kink-free radius (e.g. in the range of 0.5 to 1.5 mm) and tensile strength of at least 5N is still ensured.

According to some embodiments, the microcatheter **200** may have a length of at least 50 cm, at least 60 cm, at least 75 cm, or at least 1m. Each possibility is a separate embodiment. Each possibility is a separate embodiment.

Another optional structure of filter **220** is provided in **FIG. 4****.** According to some embodiments, the structure is suitable for a 1.7 Fr embolization microcatheter. As seen in **FIG. 4****,** filter **220** may include a plurality of side openings **225,** distributed in circumferential rings around elongated filter **220.**

According to some embodiments, filter **220** may include 2-20 or 5-15 or 6-10 rings (e.g. 9 rings) of side openings. According to some embodiments, each of the rings may include 2-10 side openings or 4-8 side openings, such as, but not limited to 6 side openings per ring. According to some embodiments, filter **220** may include a total of 30-80 or 40-60 side openings, such as but not limited to 54 side openings. According to some embodiments, the distal most of the rings of filter section 1 may be positioned about 2-10 mm or 3-6 mm, such as but not limited to about 4 mm from the distal end opening **180.**

According to some embodiments, side openings **225** may have a dimension of about 150x25 microns, about 150x30 microns, about 125x30 microns or about 100x30 microns.

According to some embodiments, each ring of side openings may be spaced apart from its neighboring ring by 500-200 microns or by 120-180 microns, such as but not limited to 150 microns.

Advantageously, filter **220** may be formed by selective cutting of the polymeric layer (leaving braid **290** essentially intact). According to some embodiments, at least some of side openings **225** may include sub-side-openings (such as sib-side-opening **225a** and **225b** in **FIG. 2D****)** separated by braid **290,** but not by the polymeric outer layer.

According to some embodiments, the slits may be positioned at a same or a different longitudinal position. Each possibility is a separate embodiment. According to some embodiments the distribution of the slits may be staggered, zig-zagged or any other suitable even or uneven distribution.

Advantageously, the filter **220** may be configured for kink-free bending despite the plurality of side openings formed in the wall thereof. According to some embodiments, the flexibility of the filter **220** is determined by the number of side openings, their minimal cross-sectional dimension, their width, length spacing, geometry, distance from distal outlet etc., as essentially described herein, may enable kink-free bending thereof.

As used herein the term "kink-free bending" may refer to a bending of filter **220,** which does impede flow therethrough. According to some embodiments, filter **220** may be configured for kink-free bending at an angle of about 180 degrees. According to some embodiments, filter **220** may be configured for kink-free bending at a minimum bending radius in the range of about 0.5 to 1.5 mm, for example 0.5 to 1.2, 0.5 to 1 mm, or any radius in-between.

Advantageously, microcatheter **200** including filter **220** provides effective reflux prevention, which requires a relatively high density of side openings, while a small kink-free radius (e.g. in the range of 0.5 to 1.5 mm) and tensile strength of at least 5N is still ensured.

According to some embodiments, the microcatheter **200** may have a length of at least 50 cm, at least 60 cm, at least 75 cm, or at least 1m. Each possibility is a separate embodiment. Each possibility is a separate embodiment.

As used herein, the terms "approximately" and "about" refer to +/-10%, or +/-5%, or +-2% vis-à-vis the range to which it refers. Each possibility is a separate embodiment.

While a number of exemplifying aspects and embodiments have been discussed above, those of skill in the art will envisage certain modifications, additions and sub-combinations thereof. It is therefore intended that the following appended claims be interpreted to include all such modifications, additions and sub-combinations as are within their scope.

## Claims

1. An embolization microcatheter (100) for embolization of peripheral arteries comprising:
an elongated tubular member (110) comprising a distal end (150) extending from a proximal marker (160) to a distal end opening (180), the distal end (150) having an outer diameter of about 0.7 mm or less,
wherein the elongated tubular member (110) comprises a multiplicity of sections, each having a length of 5 mm -120 mm, wherein a wall of each of the multiplicity of sections comprises a braid (190), a polymer formed around the braid (190) and an inner liner (192) coating an inner surface thereof, wherein the polymer of at least some of the multiplicity of sections differ; **characterized by** a thickness of the wall being less than 100 microns;
a distal tip (170) having a length of 0.5 mm - 3 mm and extending between a proximal end of a distal radiopaque marker of the elongated tubular member (110) and a distal end opening (180) of the elongated tubular member (110); and wherein the wall of the distal tip (170) is devoid of braid.

2. The embolization microcatheter (100) according to claim 1, wherein an inner lumen of the distal end (150) is in a range of 0.3 mm - 0.7 mm.

3. The embolization microcatheter (100) according to claim 1, wherein the thickness of the wall is less than 90 microns.

4. The embolization microcatheter (100) according to any one of claims 1-3, wherein the braid (190) is made of tungsten wires; or wherein the braid (190) is made of wires having a diameter of 15-20 microns; or wherein the braid (190) has a picks per inch (PPI) of 150-220.

5. The embolization microcatheter (100) according to any one of claims 1-4, wherein the multiplicity of sections comprises at least 5 sections, preferably wherein the multiplicity of sections comprises at least 9 sections.

6. The embolization microcatheter (100) according to any one of claims 1-5, wherein the distal most section of the multiplicity of sections has a length of 5-15 mm.

7. The embolization microcatheter (100) of any one of claims 1-6, wherein the distal most section of the multiplicity sections comprises a filter (220) formed in the wall of the elongated tubular member (210), the filter (220) comprising a plurality of side openings (225), the plurality of side opening (225) distributed in at least 5 circumferential rings spaced apart from each other by 100 microns - 200 microns.

8. The embolization microcatheter (100) according to claim 7, wherein the plurality of openings is in the form of axial slits, preferably wherein the axial slits have a length of about 100-150 microns and a height of about 20-40 microns.

9. The embolization microcatheter (100) according to any one of claims 7-8, wherein a distal most of the circumferential rings of the distal most filter (220) section is positioned about 2-6 mm proximally to the distal end opening (180); or wherein the plurality of side opening (225) is distributed in at least 8 circumferential rings; or wherein each of the circumferential rings comprises 4-8 axial slits; or wherein a proximal most circumferential ring of the at least 5 circumferential rings comprises fewer side openings (225) than rings distal thereto.

10. The embolization microcatheter (100) according to claim 9, wherein the proximal most circumferential ring comprises 1-3 side openings (225); or wherein the side openings (225), of the proximal most circumferential section, is circumferentially shifted relative to the side openings (225) in its neighboring circumferential section.

## Patentansprüche

1. Embolisationsmikrokatheter (100) zur Embolisation von peripheren Arterien, umfassend:
ein langgestrecktes rohrförmiges Element (110) umfassend ein distales Ende (150), das sich von einem proximalen Marker (160) zu einer distalen Endöffnung (180) erstreckt, wobei das distale Ende (150) einen Außendurchmesser von etwa 0,7 mm oder weniger aufweist,
wobei das langgestreckte rohrförmige Element (110) eine Vielzahl von Abschnitten umfasst, die jeweils eine Länge von 5 mm - 120 mm aufweisen, wobei eine Wand von jedem aus der Vielzahl von Abschnitten ein Geflecht (190), ein Polymer, das um das Geflecht (190) gebildet ist, und eine Innenauskleidung (192), die eine Innenfläche davon beschichtet, umfasst; wobei das Polymer wenigstens mancher aus der Vielzahl von Abschnitten unterschiedlich ist; **dadurch gekennzeichnet, dass** eine Dicke der Wand weniger als 100 Mikrometer beträgt;
eine distale Spitze (170), die eine Länge von 0,5 mm - 3 mm aufweist und sich zwischen einem proximalen Ende eines distalen strahlendichten Markers des langgestreckten rohrförmigen Elements (110) und einer distalen Endöffnung (180) des langgestreckten rohrförmigen Elements (110) erstreckt; und wobei die Wand der distalen Spitze (170) frei von Geflecht ist.

2. Embolisationsmikrokatheter (100) nach Anspruch 1, wobei ein inneres Lumen des distalen Endes (150) in einem Bereich von 0,3 mm - 0,7 mm liegt.

3. Embolisationsmikrokatheter (100) nach Anspruch 1, wobei die Dicke der Wand weniger als 90 Mikrometer beträgt.

4. Embolisationsmikrokatheter (100) nach einem der Ansprüche 1-3, wobei das Geflecht (190) aus Wolframdrähten besteht; oder wobei das Geflecht (190) aus Drähten mit einem Durchmesser von 15-20 Mikrometer besteht; oder wobei das Geflecht (190) 150-220 Fäden pro Inch (PPI) aufweist.

5. Embolisationsmikrokatheter (100) nach einem der Ansprüche 1-4, wobei die Vielzahl von Abschnitten wenigstens 5 Abschnitte umfasst, wobei die Vielzahl von Abschnitten vorzugsweise wenigstens 9 Abschnitte umfasst.

6. Embolisationsmikrokatheter (100) nach einem der Ansprüche 1-5, wobei der distalste Abschnitt der Vielzahl von Abschnitten eine Länge von 5-15 mm aufweist.

7. Embolisationsmikrokatheter (100) nach einem der Ansprüche 1-6, wobei der distalste Abschnitt der Vielzahl von Abschnitten einen Filter (220) umfasst, der in der Wand des langgestreckten rohrförmigen Elements (210) gebildet ist, wobei der Filter (220) eine Vielzahl von Seitenöffnungen (225) umfasst, wobei die Vielzahl von Seitenöffnungen (225) in wenigstens 5 Umfangsringen verteilt sind, die um 100 Mikrometer bis 200 Mikrometer voneinander beabstandet sind.

8. Embolisationsmikrokatheter (100) nach Anspruch 7, wobei die Vielzahl von Öffnungen in der Form von axialen Schlitzen vorliegt, wobei die axialen Schlitze vorzugsweise eine Länge von etwa 100-150 Mikrometer und eine Höhe von etwa 20-40 Mikrometer aufweisen.

9. Embolisationsmikrokatheter (100) nach einem der Ansprüche 7-8, wobei ein distaler Großteil der Umfangsringe des distalsten Filterabschnitts (220) etwa 2-6 mm proximal zu der distalen Endöffnung (180) angeordnet ist; oder wobei die Vielzahl von Seitenöffnungen (225) in wenigstens 8 Umfangsringen verteilt sind; oder wobei jeder der Umfangsringe 4-8 axiale Schlitze umfasst; oder wobei ein proximalster Umfangsring der wenigstens 5 Umfangsringe weniger Seitenöffnungen (225) als dazu distale Ringe umfasst.

10. Embolisationsmikrokatheter (100) nach Anspruch 9, wobei der proximalste Umfangsring 1-3 Seitenöffnungen (225) umfasst; oder wobei die Seitenöffnungen (225) des proximalsten Umfangsabschnitts in Umfangsrichtung relativ zu den Seitenöffnungen (225) in seinem benachbarten Umfangsabschnitt verschoben sind.

## Revendications

1. Microcathéter d'embolisation (100) pour l'embolisation d'artères périphériques comprenant :
un élément tubulaire allongé (110) comprenant une extrémité distale (150) s'étendant d'un marqueur proximal (160) à une ouverture d'extrémité distale (180), l'extrémité distale (150) ayant un diamètre externe d'environ 0,7 mm ou moins,
l'élément tubulaire allongé (110) comprenant une multiplicité de sections, chacune ayant une longueur de 5 mm à 120 mm, une paroi de chacune de la multiplicité de sections comprenant une tresse (190), un polymère formé autour de la tresse (190) et un revêtement interne (192) recouvrant une surface interne de celle-ci ; le polymère d'au moins une partie de la multiplicité de sections étant différent ; **caractérisé par** une épaisseur de la paroi inférieure à 100 microns ;
une pointe distale (170) ayant une longueur de 0,5 mm à 3 mm et s'étendant entre une extrémité proximale d'un marqueur radio-opaque distal de l'élément tubulaire allongé (110) et une ouverture d'extrémité distale (180) de l'élément tubulaire allongé (110) ; et la paroi de la pointe distale (170) étant dépourvue de tresse.

2. Microcathéter d'embolisation (100) selon la revendication 1, une lumière intérieure de l'extrémité distale (150) étant dans une plage de 0,3 mm à 0,7 mm.

3. Microcathéter d'embolisation (100) selon la revendication 1, l'épaisseur de la paroi étant inférieure à 90 microns.

4. Microcathéter d'embolisation (100) selon l'une quelconque des revendications 1 à 3, la tresse (190) étant faite de fils de tungstène ; ou la tresse (190) étant faite de fils ayant un diamètre de 15 à 20 microns ; ou la tresse (190) ayant un nombre de duites par pouce (PPI) de 150 à 220.

5. Microcathéter d'embolisation (100) selon l'une quelconque des revendications 1 à 4, la multiplicité de sections comprenant au moins 5 sections, de préférence la multiplicité de sections comprenant au moins 9 sections.

6. Microcathéter d'embolisation (100) selon l'une quelconque des revendications 1 à 5, la section la plus distale de la multiplicité de sections ayant une longueur de 5 à 15 mm.

7. Microcathéter d'embolisation (100) selon l'une quelconque des revendications 1 à 6, la section la plus distale de la multiplicité de sections comprenant un filtre (220) formé dans la paroi de l'élément tubulaire allongé (210), le filtre (220) comprenant une pluralité d'ouvertures latérales (225), la pluralité d'ouvertures latérales (225) étant réparties en au moins 5 anneaux circonférentiels espacés les uns des autres de 100 microns à 200 microns.

8. Microcathéter d'embolisation (100) selon la revendication 7, la pluralité d'ouvertures se présentant sous la forme de fentes axiales, de préférence les fentes axiales ayant une longueur d'environ 100 à 150 microns et une hauteur d'environ 20 à 40 microns.

9. Microcathéter d'embolisation (100) selon l'une quelconque des revendications 7 et 8, un anneau le plus distal des anneaux circonférentiels de la section de filtre la plus distale (220) étant positionné à environ 2 à 6 mm de manière proximale par rapport à l'ouverture d'extrémité distale (180) ; ou la pluralité d'ouvertures latérales (225) étant distribuée en au moins 8 anneaux circonférentiels ; ou chacun des anneaux circonférentiels comprenant 4 à 8 fentes axiales ; ou un anneau circonférentiel le plus proximal des au moins 5 anneaux circonférentiels comprenant moins d'ouvertures latérales (225) que des anneaux distants de ceux-ci.

10. Microcathéter d'embolisation (100) selon la revendication 9, l'anneau circonférentiel le plus proximal comprenant 1 à 3 ouvertures latérales (225) ; ou les ouvertures latérales (225), de la section circonférentielle la plus proximale, étant décalées circonférentiellement par rapport aux ouvertures latérales (225) dans sa section circonférentielle voisine.
